# EUROPEAN PATENT APPLICATION

(11) **EP 1 872 792 A1**
(43) Date of publication of application: **02.01.2008**
(21) Application number: 06116322.6
(22) Date of filing: 29.06.2006
(51) Int. Cl.: A61K 39/35, A61K 39/36

(54) **A method for the production of hydrolyzed allergen**

(71) Applicant: BioTech Tools S.A., 1120 Bruxelles (BE)
(72) Inventor: Legon, Thierry, 3360 Bierbeek (BE); Pirotton, Sabine, 1070 Bruxelles (BE); Placier, Gael, 1030 Bruxelles (BE); Kergoat, Gilles, 1030 Bruxelles (BE)
(74) Representative: von Kreisler Selting Werner

(57) **Abstract**

A purified denaturated extract of natural allergens obtainable by
a) extracting a natural source of allergens comprising allergenic proteins to form an extract,
b) purifying of said extract to remove non-protein components to form a purified extract
c) denaturating said purified extract to form a purified denaturated extract, said purified denaturated extract comprising proteins, wherein the most abundant (w/w) proteins, forming together at least 60% (w/w) of all proteins, are at least two proteins, and all proteins represent at least 60% (w/w) of the dry weight of the purified denaturated extract.

and a purified allergen hydrolysate obtainable by
a) hydrolysing a denaturated allergen to form an allergen hydrolysate,
b) purifying said allergen hydrolysate to remove peptides with a molecular weight above 10,000 Da and below 1,000 Da in order to obtain a purified hydrolysate where 70%, more preferably 80% of the peptides are between 10,000 Da and 1,000 Da.

## Description

The present invention is related to a method for the production of extracts of natural allergens, peptides from these extracts and allergen extracts obtainable by the new method.

Common allergens are pollens, house dust mites, moulds, drugs, foods and animal hair and dander.

The most common allergy diseases are rhinitis, asthma and atopic dermatitis. Allergic asthma is a chronic inflammatory disorder. Symptomatic treatment of allergic disorders is effected by use of antihistaminics, β-antogonists and corticosteroids.

Furthermore, the so called "specific" immunotherapy is based on a hyposensitization. Typically patients are administered with subcutaneous injection of the specific offending allergens. Treatment is started with small allergen doses and the doses are increased. Treatment is typically maintained for several years. This type of treatment suffers from pure patient compliance and has been questioned due to safety reasons because a patient can suffer from severe anaphylactic reactions.

In addition to methods comprising repeated subcutaneous injections there are also oral hyposensitization methods.

US 4,822,611 discloses a method for treating allergies comprising oral treatment with allergens. It describes the use of commercially available "bulk" allergenic extracts showing batch-to-batch variation and differences in extracts from different manufactures. The preparation of these extracts is not described.

GB 1 247 614 discloses a method of extracting an allergen. The aim of this method is to have a more complete and effective allergenic extract by including all extractable components of the allergen.

On the other hand, there has been a tendency to develop highly specific preparations based on single epitopes. For example, WO 00/58349 discloses an isolated and purified peptide comprising a leucin positioned two peptide bonds away from a tyrosine/arginine pair. These peptides can be used to prepare a pharmaceutical composition to accomplish treatment or prophylaxis, in this case especially directed to canine allergy in dogs.

On the one hand, methods are used to purify a specifically identified single allergenic molecule. On the other hand, people are trying to produce allergenic extracts as complete as possible.

According to the first alternative, it is always possible that the allergen preparation lacks the relevant epitopes to induce tolerance in a determined patient. The second alternative has a drawback of batch-to-batch variability and of the presence of compounds able to trigger immune response like DNA molecules, carbohydrates, lipids of complexes thereof.

It is an object of the present invention to overcome at least some of the drawbacks of prior art, especially to provide antigens from natural allergens with a significant reduced capability to trigger allergenicity reaction compared to the crude allergen extract but able to stimulate T-cells as well.

The problem is solved by the provision of methods for preparing an allergen extract comprising most of the protein containing parts of an allergen extract but with a reduced, preferably very low content of non-protein components such as nucleic acids, lipids, sugars and the like.

The extracts prepared according to the invention are superior to extracts of prior art, especially do they show a reproducible composition of proteins but are not purified to a single epitope.

The method for the production of the allergen extract of the present invention comprises the steps of
a) extracting a natural source of allergens comprising allergenic proteins to form an extract,
b) purifying of said extract to remove non-protein components to form a purified extract
c) denaturating said purified extract to form a purified denaturated extract, said purified denaturated extract comprising proteins, wherein the most abundant (w/w) proteins, forming together at least 60% (w/w) of all proteins, are at least two proteins, and all proteins represent at least 60% (w/w) of the dry weight of the purified denaturated extract.

This method is referred to as method I.

In contrast to the methods of prior art, the method of the present invention produces allergen extracts which comprise predominantly proteins without purifying the extract to a single peptide or protein.

In contrast to the products of prior art the products of the invention have following advantages:
- Immunogenic substances other than proteins are substantially removed
- The natural allergen extract is able to stimulate T-cell with the reduced ability to trigger immediate allergic reaction (basophile activation, mast cell degranulation)

As starting materials different natural occurring allergens can be used. Typical natural starting materials are milk, venom, egg, weed, grass, tree, shrub, flower, vegetable, grain, fungi, fruit, berry, nut, seed, bean, fish, shellfish, seafood, meat, spices, insect, mite, mould, animal, pigeon tick, worm, soft coral, animal dander, nematode, Hevea brasiliensis, and mixtures thereof.

After extraction of the material, the extract is purified to remove non-protein components such as sugars, lipids, nucleic acids and the like. Typical, several different proteins are present in the protein fraction of the purified extract.

According to prior art, one protein is purified and the other remaining proteins are "impurities".

In contrast thereto, it is the aim of the present invention to purify the proteins together. The relative amounts of the proteins in the purified extract can be easily measured using methods like SDS page followed by densitometry.

For 60% of total weight of the proteins, it is needed to combine the two most dominant proteins at least. More preferably, 60% of all proteins are formed by the at least 3 dominant proteins, preferably by the at least 4 dominant proteins and more preferably by at least 5, 6, 7, 8, 9 or 10 proteins.

For example, there are the following proteins:

| | |
|---|---|
| Protein 1: | 27% |
| Protein 2: | 13% |
| Protein 3: | 34% |
| Protein 4: | 19% |
| Protein 5: | 17% |

The most dominant proteins forming together at least 60% are proteins 3+1 (34+27=61%).

Furthermore, the total protein content of the purified extract is at least 60% by weight, preferably the content is at least 70% by weight or 80% by weight, more preferably 90% by weight of the purified extract.

Extraction is preferably performed with aqueous solutions. Suitable salts are salts such as but not restricted to carbonate, bicarbonate, phosphate, acetate, TRIS and HEPES.

Also in contrast to many other extraction methods, it is preferred that the amount of extraction medium is comparatively large, i.e. at least 20 times the weight of the natural source of allergens, preferably 100 time the weight or more.

Purifying of the extract may be performed by one or more of the following:
- ion exchange chromatography steps (including anion exchange chromatography and cation exchange chromatography),
- size exclusion chromatography step (also called gel filtration),
- precipitation steps,
- hydrophobic interaction chromatography steps,
- pseudo-affinity and affinity chromatographies and/or
- diafiltration.

In a preferred embodiment, at least one purification step is performed with a solution comprising one or more of a tenside and/or a denaturating agent. The tenside may be non-ionic, anionic, cationic or amphoteric. Suitable denaturating agents are chaotropic agents, reducing agents and mixtures thereof. Suitable denaturating agents are for example urea, guanidinium chloride, ethylene glycol, isopropanol. A suitable concentration of urea is 3 M or more, preferably 4 M or more. A suitable concentration of guanidinium is preferably 2 M, preferably 3 M or more. A suitable concentration of ethylene glycol and/or isopropanol is 5% or more, more preferably 10% or more, up to 20% by weight.

In some cases, the production of the purified extract according to the method I of the invention is sufficient. Extracts of this type may be used to produce ex *vivo* / *in vivo* and *in vitro* diagnosis, prophylactic and therapeutic treatment of allergic diseases. A further embodiment of the present invention is a method for the production of an allergen hydrolysate, either from extracts according to method I or from any other source. If the extract comes from any other source of purified allergens than method I, one preliminary step of denaturation is required in order to improve digestibility.

The method (method II) comprises the steps of
a) hydrolysing a denaturated allergen to form an allergen hydrolysate,
b) purifying said allergen hydrolysate to remove peptides with a molecular weight above 10,000 Da and below 1,000 Da in order to obtain a purified hydrolysate wherein 70%, more preferably 80% of the peptides are between 10,000 Da and 1,000 Da.

The advantages of the product obtained thereby are that the peptides are the digestion result of denaturated proteins. Due to a specified size calibration they have a reduced potency to induce immediate allergic reaction and pro-inflammatory reaction as well.

Denaturating, if necessary is preferably performed in the presence of chaotropic agents, reducing agents or mixtures thereof. Suitable chaotropic agents are for example urea and guanidinium chloride. Typical reducing agents are for example dithiotriethol, β-mercaptoethanol, thio-glycerol and mixtures thereof.

The hydrolysing step is typically performed with an enzyme. Suitable enzymes are for example pepsin, trypsin, chymotrypsin. This hydrolyzing step can be performed in the presence of a chaotropic agent, preferably urea or guanidinium chloride, too. During hydrolysing the concentration of urea and guanidinium chloride should be below 4 M, preferably below 3M.

In step b) of method II, peptides with a molecular weight larger than 10,000 Da or smaller than 1,000 Da, are removed.

The peptides of the purified hydrolysate, therefore, comprise peptides with a molecular weights between 1,000 and 10,000 Da. Suitable methods for removing large or small peptides are ultrafiltration and size exclusion chromatography. Again this size exclusion chromatography may be performed in the presence of a chaotropic agents, for example urea, guanidinium chloride, ethylene glycol, isopropanol and mixtures thereof.

A further embodiment of the invention is an allergen extract obtainable by methods I of the present invention. Typically also in this extract the most dominant proteins by weight, which form together at least 60% by weight of all the proteins, are at least 2 proteins, preferably at least 3 or 4 proteins or more preferred at least 5, 6, 7, 8, 9 or 10 proteins.

A further embodiment is an allergen hydrolysate obtainable by method II. It can be used for
- *in vivo* diagnosis of allergic diseases: prick tests, intracutaneous injections, conjonctival, sniff and inhalation tests
- ex *vivo* and *in vitro* diagnosis of allergic diseases: ELISA kits or standards to be used in tests
- Prophylatic and therapeutic treatments of allergic diseases: vaccine for desensitization/hyposensitization treatments and modulation of immune response with/without adjuvant combination.

The allergen extract of the present invention can be used for the preparation of a pharmaceutical composition and/or food composition for inducing tolerance. Induction of tolerance can be used to cure or prevent allergic reactions.

A further embodiment of the present invention is a pharmaceutical composition comprising the allergen extract of the present invention either in complete form or in hydrolyzed form. Additionally, pharmaceutical composition may comprise one or more of the following substances: nucleoside triphosphates, nucleoside diphosphates, nucleoside monophosphates, nucleic acids, peptide nucleic acids, nucleosides or analogs thereof, immunosuppressive cytokines, compounds inducing expression of immunoproteasomes, 1,25-dihydroxyvitamin D3 or analogs thereof, lipopolysaccharides, endotoxins, heat shock proteins, thioredoxin with either NADPH or NADP-thioredoxin reductase, dithiothreitol, adrenergic receptor agonists such as salbutanol, adrenergic receptor antagonists such as butoxamine, compounds that regulate the expression of the adhesion molecule ICAM-1, N-acetyl-L-cysteine, y-L-glutamyl-L-cysteinyl-glycine (reduced L-glutathione), alpha-2-macroglobulins, inducers for Foxp3 gene expression, flavonoids, isoflavonoids, pterocarpanoids, stilbenes such as resveratrol, tachykinin receptor antagonists, chymase inhibitors, a muco-adhesive agent for attaching the particle to the intestinal mucosal lining such as a plant lectin, zinc, zinc salts, polysaccharides, vitamins and bacterial lysates.

Based on the source of natural allergens in the composition may comprise allergens selected among milk allergens, venom allergens, egg allergens, weed allergens, grass allergens, tree allergens, shrub allergens, flower allergens, vegetable allergens, grain allergens, fungi allergens, fruit allergens, berry allergens, nut allergens, seed allergens, bean allergens, fish allergens, shellfish allergens, seafood allergens, meat allergens, spices allergens, insect allergens, mite allergens, mould allergens, animal allergens, pigeon tick allergens, worm allergens, soft coral allergens, animal dander allergens, nematode allergens, allergens of Hevea brasiliensis.

In a preferred embodiment, the pharmaceutical composition is prepared for oral administration, for sublingual drug delivery, for enteric drug delivery.
Fig. 1 : Immunoreactivity by IgG western-blot. Lane1 : molecular weight markers, lane 2 : crude protein extract, lane 3 : purified allergen denaturated extract. Membrane blocked by BSA 5 % and milk 3%. Patient serum diluted to 1/250. IgG binding was detected by goat anti-human IgG HRP diluted to 1/2,500 and revealed by TMB substrate. Allergen 1 : ± 61-54 kDa, Allergen 2: ± 36-31 kDa.
Fig. 2 : Immunoreactivity by IgE western-blot. Lane1 : molecular weight markers, lane 2 : crude protein extract, lane 3 : purified proteins. Membrane blocked by BSA 5 % and milk 3%. Patient serum diluted to 1/5. IgE binding detected by goat anti-human IgE HRP diluted to 1/10,000 and revealed by TMB substrate. Allergen 1 : ± 61-54 kDa Allergen 2: ± 36-31 kDa.
Fig. 3 : Exclusion peak of SEC G25 elution profile. The ratio column volume / sample volume was 12. The resin was equilibrated with Tris.HCl 25 mM, urea 1.5 M, pH 8.0 at a flow rate of 9 ml/min. The elution was followed by the absorbace at 280 nm.
Fig. 4 : Protein profile by SDS-PAGE. 4 - 12% Bis-Tris gel. Lane 1 : molecular weight markers, lane 2 : purified allergen denaturated extract. Staining performed with Coomassie brillant blue R-250.
Fig. 5 : Protein and peptide profiles by SDS-PAGE. 4 - 12% Bis-Tris gel. Lane 1 : molecular weight markers, lane 2 : purified allergen denaturated extract (13 µg), lane 3 : hydrolysate (13 µg). Staining performed with Coomassie brillant blue R-250.
Fig. 6 : G50 SEC elution profile. The column was equilibrated with urea 2 M, NaCl 100 mM, pH 3.0. Flow rate 15 ml/min. The ratio column volume / sample volume was 10. The elution was followed by the absorbance at 280 nm.
Fig. 7 : Calibration curve for HPLC analysis. 10 µl of the following standards (1 mg/ml) were injected onto the BioSep-SEC S2000 column: 1. Bovine Serum Albumin (66 kDa), 2. β-Lactoglobulin (18.5 kDa), 3. Cytochrome C (12 kDa), 4. Glucagon (3.5 kDa), 5. 1 kDa synthetic peptide.
Fig. 8 Size exclusion HPLC profile. Column . BioSep-SEC S2000 (PHENOMENEX). Elution buffer : Na₂HPO₄ 50 mM - SDS 0.5% (w/v) pH 6.8. Flow rate 1 ml/min. Detection at 214 nm. 10 µl of the samples were injected. The area under the curve, between 10 kDa and 1 kDa limits was used to calculate the percentage of the peptides of interest.

The method of the present invention is further exemplified by the following, nonlimiting examples.

### Examples

### Example 1: Extraction

1% (w/v) pollen *(Lolium perenne* from ALLERGON) was added to sodium bicarbonate (12.5 mM) and incubated 2 h under stirring. The solution was then clarified and filtrated by adding celite (ACROS) at 2% (w/v) and passing through a 0.2 µm filter. This sample constitutes the crude extract.

The presence of allergens in the extract was analyzed by western blotting using pollen allergic patient sera. IgG and IgE epitopes are visualised with anti-human IgG or IgE antibodies.

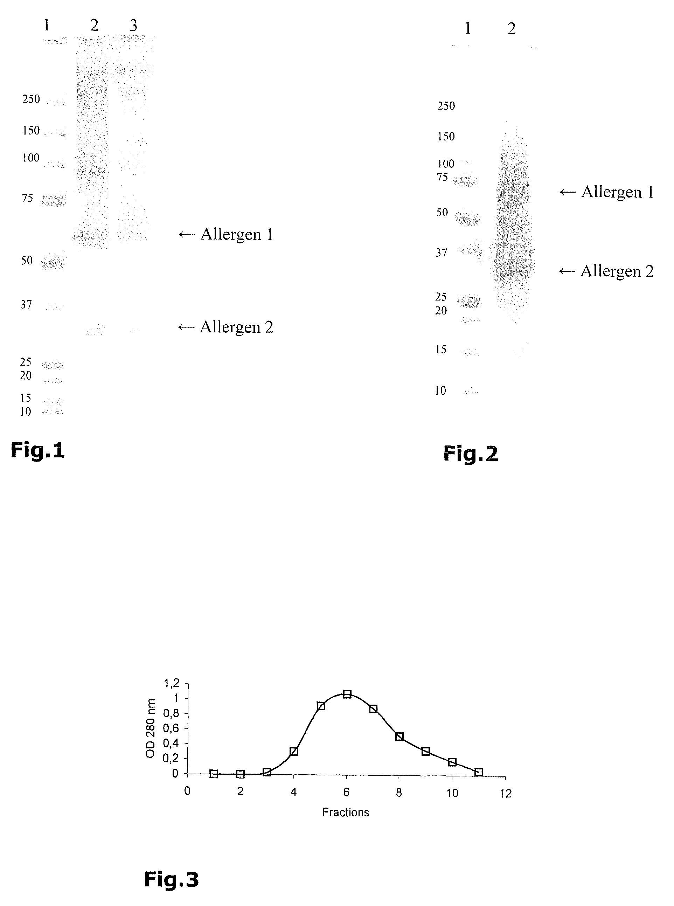

As shown on figure 1 and 2, there are two major allergens in the extract.

The said crude extract was acidified to pH 3.0 and Tween 20 (0.1%, v/v) was added. This sample constitutes the acidified extract.

### Example 2: Purification of allergen proteins

The allergen extract was purified by:
- Cation exchange chromatography
   A sartobind S- membrane (SARTORIUS) was equilibrated with 28x Bed volume (Bv) of sodium bicarbonate 12.5 mM, citrate 30 mM, pH 3.0, Tween 20 0.1% (v/v). The said acidified extract was loaded on the equilibrated membrane. The column was washed first with 35x Bv of sodium bicarbonate 12.5 mM, citrate 30 mM, pH 3.0, Tween 20 0.1% (v/v) and then washed with 42x Bv of sodium bicarbonate 12.5 mM, citrate 30 mM, pH 3.0. The proteins were eluted with carbonate 0.1 M, sodium chloride 0.5 M, pH 9.15. The presence of proteins was followed the OD at 280 nm. The fractions of interest were pooled.
- Ammonium sulfate precipitation
   This step was performed at 0-4°C.
   A quantity of ammonium sulfate to reach 90% of saturation was added to the product under stirring. The stirring was stopped after the complete dissolution of the salt. The suspension was incubated overnight and centrifuged 2 times during 15 min at 10,000 g. The supernatant was each time carefully discarded.
- Denaturation
   The pellets were resuspended at 9 mg/ml in urea 6 M, DTT 10 mM, Tris.HCl 0.1 M, pH 8.0 and incubated at 37°C for 1 h.
- Size exclusion chromatography on G25 resin (fine Sephadex from AMERSHAM)
   The denatured sample was loaded on the column and the proteins were eluted with Tris.HCl 25 mM, urea 1.5 M, pH 8.0.

The presence of proteins was followed by the OD measurement at 280 nm The fractions of interest were pooled to constitute the purifed denaturated allergen extract.

The purified allergen extract was further analysed. The protein content (BCA Assay) and the dry weight were determined in order to evaluate the protein purity. The purification efficiency was also followed by the removal of carbohydrates (Orcinol test) and by the decrease of the ratio OD₂₆₀ / OD₂₈₀.

**Table 1: Removal of non-protein components to form a purified extract**

| | Ratio protein / dry weight | Ratio OD_{260/}OD₂₈₀ | Ratio carbohydrates / proteins |
|---|---|---|---|
| Crude extract | 16% | 1.3 | 400% |
| Purified extract | 85% | 0.75 | 17% |

As shown in table 1, the purification process allows
- The increase of the percentage of proteins in the extract from ~ 15% to 80%
- The OD₂₆₀/OD₂₈₀ ratio to tends towards 0.5 characterizing a pure protein
- A significant removal of carbohydrates (the residual content could represent the carbohydrate moiety of the proteins).

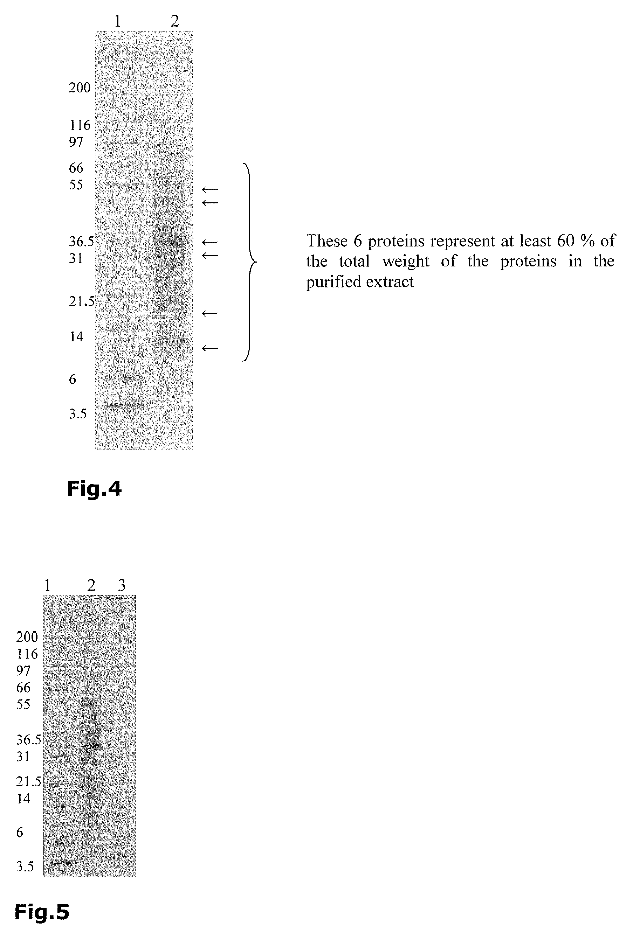

Figure 4 illustrates a typical SDS-PAGE profile obtained for the purified denaturated allergen extract. As can be seen, 6 proteins represent at least 60% of the total weight of the proteins in the purified extract.

### Example 3: Hydrolysis of denatured allergen extract

The extract was hydrolyzed using the following protocol :
The said purified allergen extract was acidified to pH 2.0. The digestion was performed at 2.5 mg/ml of pollen proteins and 1 Eu. Ph. U of pepsin (MERCK) for 337 mg of proteins, at 37°C, during 2 h.
Figure 5 shows a comparison between the purified extract (lane 2) and the hydrolyzed extract (lane 3). As can be seen, high molecular weight proteins corresponding to denatured undigested proteins have disappeared after the incubation with pepsin.

### Example 4: Purification

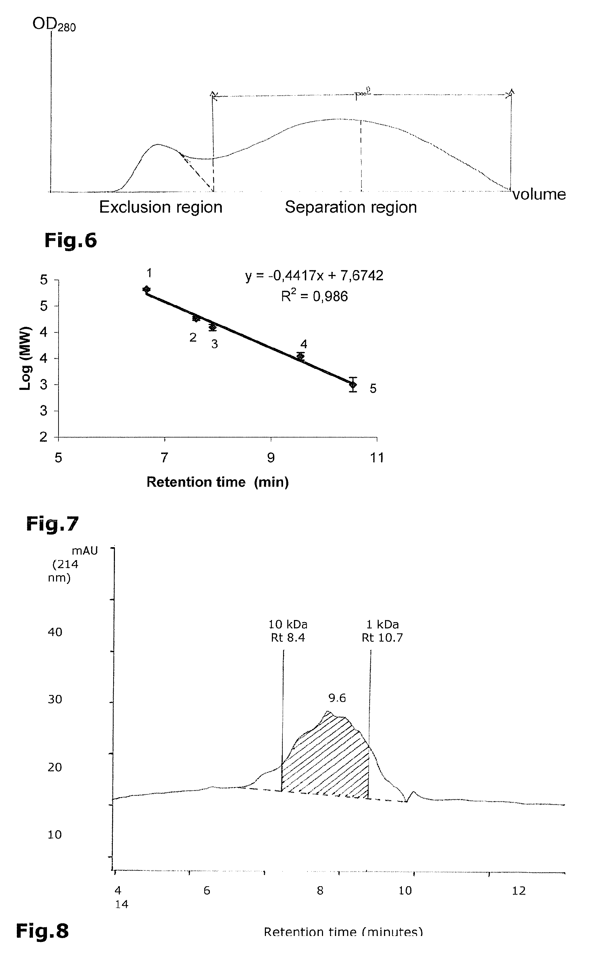

In order to eliminate the peptides with a MW ≥ 10,000 Da and MW ≤ 1,000 Da, the hydrolysate was purified by
- Size exclusion chromatography on G50 resin (fine Sephadex from AMERSHAM) 16.5 % (v/v) of isopropanol and 0.1 M of NaCl were added to the hydrolysate. This sample was immediately loaded on a G50 column. The peptides were eluted and the fractions containing the peptides (MW ≤ 10 kDa) were pooled as shown in figure 6.
- Diafiltration on 1kDa membrane (ultrafiltation cassette Omega PES from PALL) The peptides were concentrated 10 x, diafiltrated against 10 volumes of Tris.HCl 50 mM pH 7.4 and finally concentrated 2.5 x. This sample constitutes the purified allergen hydrolysate.

The efficiency of the purification was controled by size exclusion HPLC. A BioSep-SEC S2000 column (PHENOMENEX) was equilibrated with Na₂HPO₄ 50 mM - SDS 0.5% (w/v) pH 6.8 at a flow rate of 1 ml/min. The peptides were detected at 214 nm.

The 10 kDa and 1 kDa limits were calculated from a calibration curve as exemplified in figure 7.

As shown on figure 8, peptides with a molecular weight between 1,000 Da and 10,000 Da represent about 75% of all peptides in the purified hydrolysate.

## Claims

1. A purified denaturated extract of natural allergens obtainable by
a) extracting a natural source of allergens comprising allergenic proteins to form an extract,
b) purifying of said extract to remove non-protein components to form a purified extract
c) denaturating said purified extract to form a purified denaturated extract, said purified denaturated extract comprising proteins, wherein the most abundant (w/w) proteins, forming together at least 60% (w/w) of all proteins, are at least two proteins, and all proteins represent at least 60% (w/w) of the dry weight of the purified denaturated extract.

2. The extract of claim 1 wherein extracting is performed in a solution comprising no salt or a salt selected from carbonate, bicarbonate, phosphate, acetate, TRIS and HEPES.

3. The extract of claims 1 or 2 wherein extracting is performed with an extraction medium wherein the weight of the extraction medium is at least 20 times, preferably 100 times the weight of the natural source of allergens.

4. The extract of any one of claims 1 to 3 wherein the purification comprises one or more of an ion exchange chromatography step, a gel filtration or size exclusion chromatography step, a precipitation step, a hydrophobic interaction chromatography step, a pseudo affinity or affinity chromatography step or a diafiltration step.

5. The extract of any one of claims 1 to 4 wherein at least one purification step is performed with a solution comprising a tenside and/or denaturating agent.

6. The extract of claim 5 wherein denaturation is performed with a denaturating agent selected from the group of chaotropic agents, reducing agents and mixtures thereof, preferably among urea, guanidinium chloride, dithiotreitol, thioglycerol, β-mercaptoethanol and mixtures thereof.

7. The extract according to claim 6 wherein the concentration of urea is more than 4 M, preferably more than 5 M and/or the concentration of guanidinium chloride is above 3 M, preferably above 4 M.

8. A purified allergen hydrolysate obtainable by
a) hydrolysing a denaturated allergen to form an allergen hydrolysate,
b) purifying said allergen hydrolysate to remove peptides with a molecular weight above 10,000 Da and below 1,000 Da in order to obtain a purified hydrolysate where 70%, more preferably 80% of the peptides are between 10,000 Da and 1,000 Da.

9. The allergen hydrolysate of claim 8 wherein hydrolysing is performed with an enzyme, preferably pepsin, trypsin or chymotrypsin.

10. The allergen hydrolysate of any one of claims 8 to 9 wherein hydrolysing is performed in the presence of a chaotropic agent, preferably selected from urea and guanidinium chloride.

11. The allergen hydrolysate of any one of claims 8 to 10 wherein the removal of the peptides is performed by size exclusion chromatography and/or by ultrafiltration.

12. The allergen hydrolysate of claim 11 wherein the size exclusion chromatography step is performed in the presence of a chaotropic agent, preferably selected among urea, guanidinium chloride, ethylene glycol, isopropanol and mixtures thereof.

13. The allergen hydrolysate of any one of claims 8 to 12, wherein, prior to hydrolysis, a not denaturated allergen is denaturated to form a denaturated allergen.

14. The allergen hydrolysate of claim 13 wherein denaturating is performed in the presence of chaotropic agents, reducing agents and mixtures thereof.

15. A method for the production of a purified extract of natural allergens comprising the steps of
a) extracting a natural source of allergens comprising allergenic proteins to form an extract,
b) purifying of said extract to remove non-protein components to form a purified extract
c) denaturating said purified extract to from a purified denaturated extract, said purified denaturated extract comprising proteins, wherein the most abundant (w/w) proteins, forming together at least 60% (w/w) of all proteins, are at least two proteins, and all proteins represent at least 60% (w/w) of the dry weight of the purified denaturated extract.

16. A method for the production of a purified allergen hydrolysate comprising the steps of
a) hydrolysing a denaturated allergen to form an allergen hydrolysate,
b) purifying said allergen hydrolysate to remove peptides with a molecular weight above 10,000 Da and below 1,000 Da in order to obtain an allergen hydrolysate wherein 70%, more preferably 80% of the peptides are between 10,000 Da and 1,000 Da.

17. Use of the allergen extract of claims 1 to 7 or the allergen hydrolysate of claims 8 to 14 for the preparation of a pharmaceutical composition and/or food composition for inducing tolerance.

18. The use of claim 17 wherein induction of tolerance is used to cure or prevent allergic reactions.

19. The pharmaceutical composition comprising an allergen extract according to claims 1 to 7 or an allergen hydrolysate of claims 8 to 14.

20. The pharmaceutical composition of claim 19 comprising additionally at least one substance selected from the group of nucleoside triphosphates, nucleoside diphosphates, nucleoside monophosphates, nucleic acids, peptide nucleic acids, nucleosides or analogs thereof, immunosuppressive cytokines, compounds inducing expression of immunoproteasomes, 1,25-dihydroxyvitamin D3 or analogs thereof, lipopolysaccharides, endotoxins, heat shock proteins, thioredoxin with either NADPH or NADP-thioredoxin reductase, dithiothreitol, adrenergic receptor agonists such as salbutanol, adrenergic receptor antagonists such as butoxamine, compounds that regulate the expression of the adhesion molecule ICAM-1, N-acetyl-L-cysteine, y-L-glutamyl-L-cysteinyl-glycine (reduced L-glutathione), alpha-2-macroglobulins, inducers for Foxp3 gene expression, flavonoids, isoflavonoids, pterocarpanoids, stilbenes such as resveratrol, tachykinin receptor antagonists, chymase inhibitors, a muco-adhesive agent for attaching the particle to the intestinal mucosal lining such as a plant lectin, zinc, zinc salts, polysaccharides, vitamins and bacterial lysates.

21. A pharmaceutical composition of claims 19 or 20 wherein the allergens are selected among milk allergens, venom allergens, egg allergens, weed allergens, grass allergens, tree allergens, shrub allergens, flower allergens, vegetable allergens, grain allergens, fungi allergens, fruit allergens, berry allergens, nut allergens, seed allergens, bean allergens, fish allergens, shellfish allergens, seafood allergens, meat allergens, spices allergens, insect allergens, mite allergens, mould allergens, animal allergens, pigeon tick allergens, worm allergens, soft coral allergens, animal dander allergens, nematode allergens, allergens of Hevea brasiliensis.

22. A pharmaceutical composition according to anyone of claims 19 to 21 for oral administration, for sublingual drug delivery, for enteric drug delivery.
